# EUROPEAN PATENT APPLICATION

(11) **EP 1 942 345 A1**
(43) Date of publication of application: **09.07.2008**
(21) Application number: 07000202.7
(22) Date of filing: 05.01.2007
(51) Int. Cl.: G01N 33/497, A61B 5/097

(54) **Self-purging, air-stabilizing, illuminated collection system for breath analysis**

(71) Applicant: KHN Solutions LLC, San Francisco CA 94133 (US)
(72) Inventor: Shew, David, San Francisco CA 94133 (US); Nothacker, Keith, San Francisco CA 94133 (US)
(74) Representative: Zimmermann, Tankred Klaus

(57) **Abstract**

A breath analysis system includes a dual-entry, illuminated breath collection tube that allows purging of ambient air while stabilizing the pressure and/or turbulence of exhaled breath. Circular illumination at either end of the collection tube acts as a visual prompt for proper use and for the reporting of test results. The collection tube may be placed along the width or length of a hand piece without adding size or bulk to the hand piece. The form factor utilization feature of the collection tube is suitable for adding breath alcohol measurement or other breath measurement capability to handheld products such as cell phones, PDAs, iPods^{®}, MP3 players, GPS units, cigarette lighters, and police batons.

## Description

### FIELD OF THE INVENTION

This invention relates generally to breath alcohol measuring devices and other breath measurement devices and discloses a new method and new apparatus for breath collection and measurement utilizing the form factor of portable handheld devices and in one embodiment provides a breath analysis system comprising a portable dual-entry, self-purging, air-stabilizing, illuminated breath collection tube.

### BACKGROUND OF THE INVENTION

Breath collection and breath analysis are extremely important tools, for example, in the battle against drunk driving and other inappropriate behavior exhibited by intoxicated people. The ability to test breath in an efficient manner is paramount in curtailing the excessive use of alcohol. In order to be most effective, a breath alcohol measuring device must be portable, readily available, accurate, sanitary, and unobtrusive in use and storage.

Most current breath alcohol measuring devices fall into two categories, the first being "open flow" in which breath is blown into a breath testing unit without lip or mouth contact, and the second being "closed flow" in which a user's mouth or lips touch a straw or mouthpiece connected to the breath testing unit.

On the one hand, an advantage of an open flow device is that subsequent users are not faced with the dilemma of cleaning or replacing a mouthpiece. However, typical open flow devices position a sensor behind a protective grill, leaving the sensor exposed to ambient air and subjecting the sensor to varying volumes of turbulent breath. Unfortunately, these typical open flow devices fail to stabilize breath flow prior to breath alcohol analysis by a sensor. Where a sensor is exposed to variable volumes of breath, fine calibration of the sensor is not possible, which leads to inaccurate measurements.

On the other hand, closed flow devices typically use a straw or long mouthpiece to stabilize and/or monitor breath flow and have a disposable mouthpiece or a disposable protective cover over the mouthpiece to provide for the sanitation of the device, for example. A closed flow device commercially available from Lifeloc Technologies, Inc. has a detachable straw and is used by professional law enforcement for preliminary screenings. Closed flow devices typically have greater accuracy than open flow units since the closed flow devices are able to stabilize and monitor breath prior to testing by a sensor. Unfortunately, closed flow devices are less practical as they require constant cleaning or part replacement.

Other breath collection devices known in the art have only one breath collection orifice and fail to provide efficient means for purging ambient air and/or old breath. The present invention is a departure from the related art by its disclosure of various embodiments comprising a unique self-purging breath collection tube for breathing into without lip or mouth contact with the tube. Various embodiments also comprise a dual-entry collection tube to allow users the convenience of blowing breath into either end of the collection tube. The dual-entry feature of the disclosed collection tube allows the unit to facilitate self-purge as ambient air and/or old breath is pushed out as a user blows into either end of the tube.

The disclosed breath analysis system may be used in an open flow manner but yet results in very accurate measurements as breath is stabilized as it flows through the breath collection tube prior to reaching an internal sensor.

The disclosed breath analysis system may be installed along the length or width of a handheld device, taking advantage of the existing form factor found in most handheld devices. For example, the disclosed breath analysis method and apparatus may be incorporated into dedicated handheld alcohol detection devices or into a myriad of handheld devices such as portable phones (for example, cell phones), music players (for example, iPods^{®}), Personal Digital Assistants (PDAs), Global Positioning System (GPS) units, car key holders, police batons, flashlights, and other portable items, for example. The disclosed breath collection tube may be placed along the length or width of a handheld device such as a phone unit to inconspicuously add the purge feature and breath stabilization feature needed for more accurate breath testing.

### DESCRIPTION OF THE RELATED ART

Published U.S. Patent Application No. 20050053523 by Brooke shows a combination alcohol tester/cell phone unit but fails to provide means to stabilize or monitor the flow of exhaled breath that reaches the sensor of the unit. The Brooke application also fails to teach a method of purging ambient air and old breath from the breath testing portion of the unit.

The Brooke application merely shows an alcohol sensor behind a grill. Such a design leads to inaccurate testing, as the Brooke sensor is exposed to variable breath pressure and ambient air and/or old breath from prior users.

Patent No. 5,361,772 by Murnick discusses the benefits of purging ambient air out of a breath collection device and discloses a single entry and single use breath collection tube. The Murnick breath collection tube has two open ends wherein ambient air is purged as a user exhales into one designated end of the tube, pushing ambient air out through the opposite end of the tube. Unfortunately, the Murnick collection tube merely captures a stream of expired breath and requires the use of a separate testing machine to analyze a user's breath. The Murnick collection tube merely seals a breath sample and is not suitable for field use, as a separate collection tube is required to capture and store each breath sample.

Patent No. 4,744,953 by Wolf discloses a portable breath alcohol detector with a mouthpiece that acts as a whistle to monitor breath flow. The Wolf whistle allows a user to regulate breath input by listening to the whistle. The Wolf whistle is a closed flow device, and requires a user to place his or her lips on a tubular mouthpiece, thereby compromising the sanitation of the device. The sound generated by the Wolf whistle prevents discreet use, use in noisy environments, or use by the hearing impaired.

### BRIEF SUMMARY OF THE INVENTION

An object of this invention is to provide a method and apparatus for breath alcohol and other breath content detection that is highly accurate, requires no lip or mouth contact, is discreet, and can be incorporated into small dedicated handheld units or handheld products such as cell phones, iPods^{®}, PDAs, GPS units, police batons, directional batons, and all other handheld devices. This invention presents a reusable handheld device that solves several problems in the related art by disclosing a new method and new apparatus to purge ambient air and old breath and to stabilize exhaled breath. The disclosed breath collection tube may be placed along the length or width of a handheld device such as a phone unit to inconspicuously add the purge feature and breath stabilization feature needed for more accurate breath testing.

In one embodiment, a dual-entry, self-purging, air-stabilizing, illuminated, breath collection method and apparatus provide accurate alcohol detection without the need for lip or mouth contact. The related art requires the use of mouthpieces to create a steady stream of air. Such mouthpieces require cleaning or replacement after each use, and are not conducive to use among a group of people in social settings such as parties where alcohol consumption often occurs. The related art that does not require lip or mouth contact fails to provide accurate results, because the sensors receive variable amounts of breath where a user simply blows directly on a sensor or on a sensor placed behind a grill.

In one embodiment, a dual-entry breath collection tube is incorporated that spans from one end of a hand piece to the other. By taking advantage of the native dimensions of the hand piece, the disclosed dual-entry collection tube may be used without adding bulk or size to the hand piece.

The dual-entry breath collection tube is open on both ends, allowing ambient air and old breath to be purged during use, which ensures that ambient air and breath from a prior user are exhausted from the collection tube. The collection tube stabilizes exhaled breath and provides consistent breath pressure and volume to an internal sensor(s), allowing for greater accuracy and precision in the testing of breath samples. The internal sensor(s) may be used to detect breath alcohol levels, bad breath, or other substances in the breath.

The dual-entry feature of the breath collection tube is unknown in the prior art and facilitates alcohol detection by multiple users. The breath analysis system may be passed from one user to another user without delay needed for cleaning or mouthpiece replacement. A subsequent user may exhale into the collection tube end opposite to that used by the previous user or, if desired, exhale into the same end of the collection tube used by the prior user. The ability to blow through either end of the collection tube allows for rapid use by multiple users, as the breath analysis system does not need to be rotated after a typical hand-to-hand exchange.

In one embodiment, a new method of passing a breath analysis system is provided. The breath analysis system may be passed to a subsequent user without rotation. Thus, a subsequent user is presented with the end of the collection tube not blown into by the prior user. This new method of passing a breath collection system allows for faster breath testing among a group of people.

In one embodiment, a source of illumination is incorporated. The use of illumination at either end of the collection tube allows users to visually monitor the flow and volume of exhaled breath entering the breath analysis system. The illumination may act as an instant visual aid to allow a user to adjust his or her exhalation to meet the performance criteria selected by the user or set by the manufacturer. Users wishing to increase the accuracy of a breath test may preferably adjust the breath analysis system to require a greater volume of exhaled breath. Those skilled in the art will recognize that a greater volume of breath will generate a greater amount of alveolar air, which may result in a more accurate measure of alcohol intoxication, for example.

The disclosed breath analysis system positions a sensor or plurality of sensors in the middle of a breath collection tube or in a sensor airway tube which is connected to the collection tube. The internal placement of sensors solves problems in the related art due to sensor placement behind a mere grill such as disclosed in the Brooke application.

The disclosed breath analysis system preferably incorporates a dual-entry breath collection and purge tube with the added benefit of instant, on-site measurement of breath content, a function not possible with the Murnick collection tube. Unlike the Murnick collection tube, the disclosed breath analysis system uses a collection tube to purge air, stabilizes a breath sample, and achieves an accurate and immediate analysis of breath content. The disclosed breath analysis system may be passed from user to user and does not require lip or mouth contact. Unlike the Murnick collection tube, the disclosed breath analysis system provides instant analysis of breath, without the need for a separate apparatus to empty a collection tube. Unlike the Murnick collection tube, the tube may be blown into at either end.

The disclosed breath analysis system overcomes the shortfalls of the Wolf whistle by allowing users to monitor breath flow in a discreet manner and without the need for lip or mouth contact. Users of the disclosed breath analysis system may view lights placed at the circumference of either end of the breath collection tube. The lights inform the user if proper breath pressure and/or volume are present and may report test results. The illuminating feature also gives the user a visual target. Unlike the Wolf whistle, the disclosed system may be used without cleaning, in the dark, and in both noisy and quiet settings.

Other objectives of the present invention will become apparent to those skilled in the art in light of the following description and disclosed drawing.

### BRIEF DESCRIPTION OF THE SEVERAL VIEWS OF THE DRAWING

FIG. 1 is a front cross-sectional view showing the dual-entry, self-purging, air-stabilizing breath collection tube and the attached airway leading to a sensor in accordance with one embodiment of the present invention.

FIG. 2 is a side view showing a ring of illumination at the outer circumference of the breath collection tube shown in FIG. 1.

FIG. 3 is a front view of a handheld unit containing the breath collection tube, a start button, and a flat visual display on the face of the unit.

FIG. 4 is a front cross-sectional view showing a bowed collection tube that has a greater interior diameter at the ends and a lesser interior diameter in the middle region.

### DETAILED DESCRIPTION OF THE PREFERRED EMBODIMENTS OF THE INVENTION

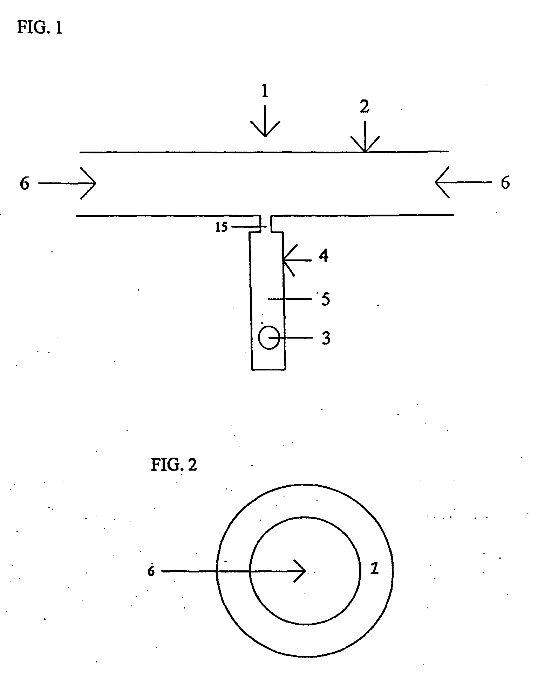

Referring to the drawing for one of many illustrative embodiments of the present invention, FIG. 1 shows a preferred embodiment of a dual-entry, self-purging, air-stabilizing breath analysis system 1 including a breath collection tube 2. The overall length of the collection tube 2 may be approximately 5 cm. The two ends 6 of the collection tube 2 are both suitable for breath entry. Thus, a user may blow into either end 6 of the collection tube 2 from a distance without contacting the collection tube with his or her lips or mouth as shown in FIG. 1, and ambient air and/or old breath will purge though the opposite end.

FIG. 1 shows the dual-entry, breath collection tube 2 connected to a sensing device 4 comprising a sensor airway 5. Within the sensor airway 5, a sensor 3 is positioned to analyze the contents of breath. For example, the sensor 3 may be a model number SB-EN2 gas sensor for alcohol detection commercially available from FIS Inc. It is a tindioxide semiconductor gas sensor. Alternatively, the sensor 3 may additionally or alternatively be a model number SB-AQ4 sensor commercially available from FIS Inc. to detect cigarette smoke or a sensor to test for gases associated with bad breath such as a model number SB-EN3 sensor commercially available from FIS Inc. A full product list of FIS Inc. sensors can be viewed at http://www.fisinc.co.jp/04_prod.htm.

Preferably, as shown in FIG. 1, a region 15 may be an air passage of approximately 1.5 mm in diameter. The remainder of sensor airway 5 of the sensing device 4 may be approximately 5 mm in diameter. The constricted region 15 of the sensor airway 5 may be referred to as a "pinhole" airway connecting to the collection tube 2.

By travel through the collection tube 2, exhaled breath is stabilized prior to reaching the attached sensing device 4. The region 15 of the sensor airway 5 has a smaller cross-section than the remaining portion of the sensor airway 5. The connected sensor airway 5 further stabilizes exhaled breath prior to contact with the internal sensor 3.

In accordance with various contemplated modifications of the embodiment of the invention shown in FIG. 1, various sensor(s) may be contained in the breath collection tube 2 and/or in the attached sensor airway 5. The sensor 3 may also be replaced with a plurality of sensors.

The interior of the attached sensor airway 5 may also contain means to measure and/or change breath pressure and/or breath temperature.

In accordance with one embodiment, illumination of the circumference of the ends of collection tube 2 may be provided, as shown at 7 in FIG. 2. The area of illumination 7 at either end of the collection tube 2 may be circular, as shown in FIG. 2, or may be of any other shape that forms around the circumference of the collection tube. The ring of illumination 7 may simply be a translucent piece of plastic with the actual LED bulbs mounted on an internal circuit board and light piped to the ring.

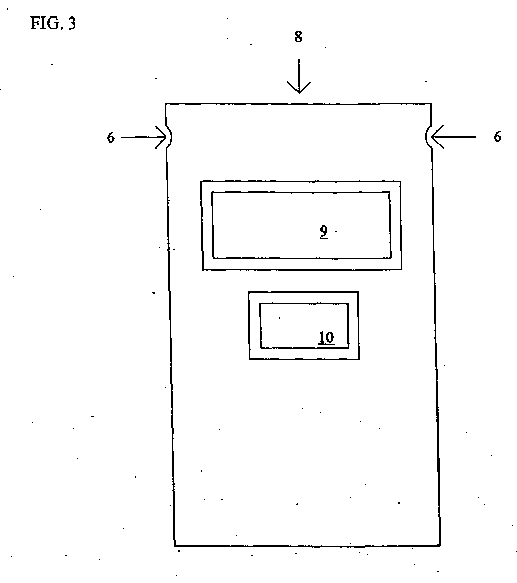

Preferably, a ring of illumination 7 at either end of the collection tube 2 may serve as a target for directing exhaled air into the end 6 of the collection tube. Before a test, there is typically a countdown to heat the sensor and prepare the breath analysis system before the user blows into the collection tube 2. The ring of illumination 7 can either blink or turn on or off to signify different modes and when the system is ready to start a test. Users can simply follow the visual prompting of the illumination 7 to tell them when to start blowing and for how long. This visual prompt cue interface coupled with an audible beep/chime sound from a speaker, as well as a flat panel display 9 shown in FIG. 3, provide user guidance. In accordance with various contemplated embodiments, the illumination 7 may vary to indicate proper breath pressure, breath volume, and to report test results. Such information may also be transmitted by a separate visual display located on the hand piece such as a flat panel display 9, for example, a backlit liquid crystal display panel, as shown in FIG 3. The visual display panel 9 shown in FIG. 3 may use color to add emphasis to test results. For example, green may be used to show a safe level of breath alcohol, and red may be used to warn of dangerous intoxication.

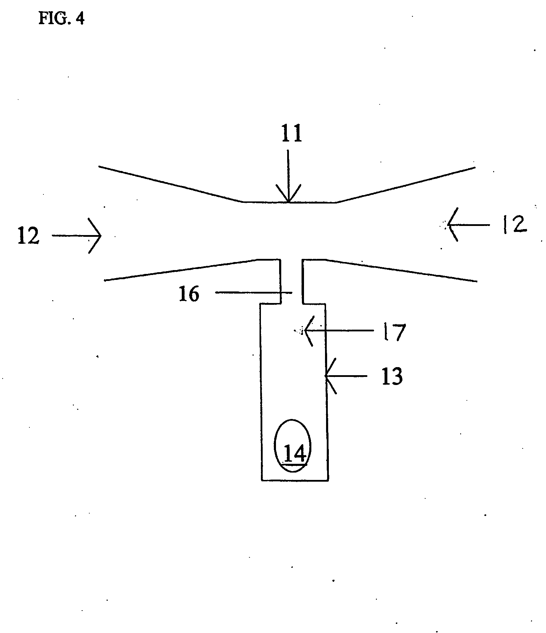

FIG.4 shows an alternative embodiment of a breath collection tube 11. The inside diameters of the two larger ends 12 of the collection tube 11 may be approximately 7 mm. The inside diameter at the middle area of the collection tube 11 may be approximately 5 mm.

The tapered or bowed breath collection tube 11 shown in FIG. 4 may be attached to a sensing device 13 comprising a sensor airway 17. A region 16 of the sensor airway 17 connected to the collection tube 11 may be smaller in cross-section than the lower portion, which contains a sensor 14.

The breath collection tube 2 or 11 may be round or may take the form of other shapes, such as a square tube.

The apparatus and method in accordance with the various embodiments of the present invention may be incorporated into a dedicated handheld device such as shown at 8 in FIG. 3 or incorporated into a myriad of self-powered handheld units such as police batons, traffic sticks, portable phones, iPods^{®}, portable music players, portable Dictaphones, computers, calculators, car key holders, karaoke microphones, and all other tangible items. The method and apparatus in accordance with the present invention may also be incorporated into any handheld consumer device.

This invention allows for individuals and a group of people to check levels of alcohol consumption, for example, in a fast, discreet, and efficient manner, allowing for timely decisions regarding present and future conduct.

The illumination area 7 shown in FIG. 2 may serve to regulate user compliance during breath exhalation. A user may view the illumination and accordingly adjust breathing to optimal levels during the test. Unlike the related art, the breath analysis system in accordance with the various embodiments of the present invention allows a user to make breathing adjustments during the testing phase and does not require the user to abandon an attempt due to inadequate exhalation. The breath analysis system does not require the user to estimate the proper exhalation input, as the illumination preferably communicates current breath pressure data. The illumination area 7 shown in FIG.2 may serve to indicate readiness of the breath analysis system, as there may be a time delay between pushing a start button 10 shown in FIG. 3, and the sensor 3 or 13 being heated to an optimal testing temperature.

Various embodiments of the present invention allow breath to be blown into either end of the breath collection tube 2 or 11. Thus, users are able to "hand off" the breath analysis system without the burden of rotating the system to find the proper input orifice.

The dual-entry feature of the breath collection tube 2 or 11 provides a unique advantage, as breath may be blown into either end of the collection tube. The end of the collection tube 2 or 11 opposite the user allows ambient air and/or old breath to purge from the collection tube.

The illumination 7 at either end of the collection tube 2 or 11 may provide information to the user during all stages of testing. Different colors and different patterns of illumination may be used to indicate readiness for use, remaining duration for the current period of exhalation, required user adjustments to exhalation during the test period, sufficiency of exhalation, and test results. Such information may also be displayed on the exterior of the breath analysis system in other visual displays. In accordance with a contemplated modification such information may be additionally or alternatively communicated to the user in an audio message.

The preferred sequence of operation of the breath analysis system for breath alcohol testing is as follows.
1) A user presses the power button 10 to turn on the system.
2) An audible "beep" and the rings of illumination 7 turn on.
3) The system counts down for approximately 10 seconds. During this time, the sensor 3 or 13 is being heated.
4) When the system is ready for a breath sample, rings of illumination 7 flash two times, the audible beep sounds, and the LCD screen 9 displays "blow".
5) The user begins blowing into the breath collection tube 2 or 11.
6) After the system determines a sufficient lung air sample has been blown by the user, rings of illumination 7 flash three times, and another audible beep sounds to instruct the user to stop blowing.
7) The user's breath alcohol level is displayed by the LCD screen 9.
8) The user can turn off the system by pressing the power button 10, or begin another test by pressing the power button 10 twice. If no action is taken after the initial test, the system turns off automatically after 20 seconds, for example.
9) If the system determines not enough air has been blown, rings of illumination 7 do not flash, and the LCD screen 9 displays "ERR" and the user must press the power button 10 to turn the system off and on again to retest.

It is contemplated that the breath analysis system may have two separate LED lights including a red light piped through a light tube if the user does not blow a sufficient lung air sample, and a green light if the test is performed properly. The lights could also flash red if the user's alcohol level is determined to be an unsafe level, for example, above 0.08%.

In accordance with other contemplated embodiments of the present invention, the breath analysis system may also include means to measure and adjust exhalation pressure and temperature to further increase the accuracy and precision of breath measurement. Sensors connected to a computer controlled unit may be placed in the breath collection tube 2 or 11 and/or the attached sensor airway 4 or 13 to measure air pressure, temperature, and total air volume for the test period. A computer controlled unit may then operate valves or heating elements or other means to adjust breath to be tested by a sensor or plurality of sensors located throughout the system. The sensors 3 and 13 may measure alcohol content or other substances.

While the foregoing description has been with reference to particular embodiments of the present invention, it will be appreciated by those skilled in the art that changes in these embodiments may be made without departing from the principles and spirit of the invention. Accordingly, the scope of the present invention can only be ascertained with reference to the appended claims.

## Claims

1. A breath analysis system, comprising:
a breath collection tube having at least a first end into which breath is exhaled by a user without lip or mouth contact with the first end and a second end from which air is exhausted to self-purge the system, the breath collection tube for stabilizing the flow of exhaled breath; and
a sensing device comprising:
a sensor airway; and
at least one sensor.

2. The breath analysis system of claim 1 wherein the breath collection tube is tapered from the first end to a location intermediate the first and second ends.

3. The breath analysis system of claim 1 wherein the breath collection tube is a dual-entry breath collection tube in which breath is exhaled by the user into either the first or second end without lip or mouth contact with the first or second end and air is exhausted from the second or first end, respectively.

4. The breath analysis system of claim 3 wherein the breath collection tube is tapered from the first end and the second end to a location intermediate the first and second ends.

5. The breath analysis system of claim 1 wherein the sensing device is positioned at substantially the center of the breath collection tube and breath is measured at the center portion of the collection tube by the sensor or plurality of sensors capable of measuring content of breath.

6. The breath analysis system of claim 5 wherein the center of the breath collection tube connects to the sensor airway directing breath to the sensor or plurality of sensors capable of measuring the content of breath.

7. The breath analysis system of claim 1 wherein the sensor airway comprises a first region forming a pinhole connected to the breath collection tube and a contiguous remaining portion having a relatively larger cross-section, in which breath passes through a the pinhole and then passes through the remaining larger portion of the sensor airway.

8. The breath analysis system of claim 1 wherein the temperature of breath is measured.

9. The breath analysis system of claim 1 wherein the volume of breath is measured.

10. The breath analysis system of claim 8 wherein the temperature of breath is adjusted prior to reaching the sensor.

11. The breath analysis system of claim 9 wherein the volume of breath is adjusted prior to reaching the sensor.

12. The breath analysis system of claim 1, further comprising illumination at the first end of the breath collection tube to assist the user.

13. The breath analysis system of claim 12 wherein the illumination reports test results.

14. The breath analysis system of claim 3, further comprising illumination at the first and second ends of the breath collection tube to assist the user.

15. The breath analysis system of claim 14 wherein the illumination reports test results.

16. The breath analysis system of claim 1 wherein the breath collection tube is positioned internally along the length of a handheld device.

17. The breath analysis system of claim 1 wherein the breath collection tube is placed internally along the width of a handheld device.

18. The breath analysis system of claim 1 wherein the breath collection tube is placed in a mobile phone, GPS unit, portable music player, or other handheld device.

19. The breath analysis system of claim 1 wherein the sensor is capable of measuring alcohol intoxication.

20. The breath analysis system of claim 1 wherein the sensor is capable of measuring the content of breath.

21. The breath analysis system of claim 1 wherein the sensor is capable of measuring one or more indicia of unpleasant breath.

22. A method for performing breath analysis, comprising the steps of:
providing a breath collection tube having at least a first end and a second end;
a sensor for measuring a characteristic of exhaled breath;
exhaling breath into the first end of the breath collection tube without lip or mouth contact with the first end;
exhausting air from the second end of the breath collection tube to purge at least one of ambient air and old breath;
stabilizing the flow of exhaled air within the breath collection tube; and
channeling at least a portion of the exhaled air to the sensor.

23. The breath analysis method of claim 22 wherein the breath collection tube is a dual-entry breath collection tube in which breath is exhaled into either the first or second end without lip or mouth contact with the first or second end and air is exhausted from the second or first end, respectively.

24. The breath analysis method of claim 22 wherein the sensor is located in a middle portion of the breath collection tube.

25. The breath analysis method of claim 22, further comprising the step of providing a sensing device having a sensor airway connected to the breath collection tube and containing the sensor.

26. The breath analysis method of claim 25 wherein the sensor airway is narrower at an end connected to the breath collection tube and wider toward an opposite end.

27. The breath analysis method of claim 22, further comprising the step of illuminating the first end of the of the breath collection tube.

28. The breath analysis method of claim 27, further comprising the step of adjusting the illumination to report breath input.

29. The breath analysis method of claim 27, further comprising the step of adjusting the illumination to report test results.

30. The breath analysis method of claim 28 wherein the illumination reports breath input during a test period.

31. The breath analysis method of claim 22, further comprising the step of adjusting the volume of breath required for a test sample.

32. The breath analysis method of claim 31 wherein the volume of breath that reaches the sensor is adjusted.

33. The breath analysis method of claim 22, further comprising the step of measuring the temperature of exhaled breath.

34. The breath analysis method of claim 33, further comprising the step of adjusting the temperature of exhaled breath.

35. The method of handling a hand piece containing the breath analysis system of claim 3 wherein a prior user hands the hand piece directly to a subsequent user, maintaining the orientation of the hand piece such that the subsequent user is presented with the breath collection tube end not breathed into by the prior user.
